# EUROPÄISCHE PATENTANMELDUNG

(11) **EP 1 245 669 A1**
(43) Veröffentlichungstag der Anmeldung: **02.10.2002**
(21) Anmeldenummer: 02004734.6
(22) Anmeldetag: 01.03.2002
(51) Int. Cl.: C12M 3/00, C12N 13/00, C12N 15/02

(54) **Kammer zur Behandlung von in einer Suspension enthaltenen Zellen im elektrischen Feld**

(30) Priorität: 27.03.2001 DE 10116211
(71) Anmelder: EPPENDORF AG, 22339 Hamburg (DE)
(72) Erfinder: v. Beichmann, Boris, 22391 Hamburg (DE); Faustmann, Olaf, Dr., 21641 Apensen (DE); Lucas, Kurt, 22359 Hamburg (DE); Taesler, Christian, Dr., 21614 Buxtehude (DE); Gülzow, Nico, 22337 Hamburg (DE); Lübker, Wolfgang, 22851 Norderstedt (DE); Pavlovic, Nada, Dr., 22761 Hamburg (DE); Ricklefs, Hans-Joachim, 22941 Bargteheide (DE)
(74) Vertreter: Emmel, Thomas, Dipl.-Biol., Dr.

(57) **Zusammenfassung**

Kammer zur Behandlung von in einer Suspension enthaltenen Zellen im elektrischen Feld, mit einer Aufnahme, in der die Suspension angeordnet werden kann und mit mindestens zwei Elektroden, deren Elektrodenflächen einander zugewandt ausgerichtet sind, wobei zwischen den Elektrodenflächen Suspension anordenbar ist, und die Elektroden mit unterschiedlichen Polen einer Spannungsquelle zur Erzeugung eines elektrischen Feldes zwischen den Elektrodenflächen verbindbar sind und wobei die Elektroden so beschaffen sind, daß ein inhomogenes Feld erzeugbar ist, ist dadurch gekennzeichnet, daß die Elektroden plattenförmig und geschlossenflächig ausgebildet sind und Elektrodenflächen aus elektrisch leitendem Material aufweisen, wobei die Elektrodenfläche mindestens einer der beiden Elektroden so geformt ist, daß zwischen beiden Elektroden ein inhomogenes Feld mit mehreren über die Elektrodenfläche verteilten Maxima erzeugbar ist.

## Beschreibung

Die Erfindung bezieht sich auf eine Kammer nach dem Oberbegriff des Anspruches 1, insbesondere auf eine Kammer, die zur Elektrofusion von Zellen eingesetzt werden kann.

Bei der Elektrofusion werden in aller Regel jeweils zwei unterschiedliche Zellen in einem elektrischen Feld miteinander fusioniert. Der Begriff "Zellen", soweit er im Rahmen dieser Anmeldung verwendet wird, soll sich generell auf biologische Zellen beziehen, insbesondere auf solche aus Körpergeweben.

Zur Zeit von besonderem Interesse ist z.B. die Herstellung von Fusionsprodukten aus sogenannten dendritischen Zellen und Tumorzellen mittels Elektrofusion. Dendritische Zellen sind immunoaktive Zellen, die auf ihrer Oberfläche Antigene präsentieren können und in Abhängigkeit von den Antigenen unterschiedliche immunostimulierende Eigenschaften aufweisen.

Fusionsprodukte aus dendritischen Zellen und Tumorzellen können z.B. zum Einsatz bei der Therapie von Tumoren kommen. Es wird in diesem Zusammenhang auf die Veröffentlichung von Kugler et. al. in Nature Medicine Vol. 6 Seite 332 bis 336 (2000) verwiesen, die den Einsatz der erwähnten Fusionsprodukte bei der Behandlung von Nierenkarzinoma beim Menschen beschreibt.

Gattungsgemäße Kammern (auch Fusionskuvetten genannt), in denen die Elektrofusion von Zellen durchgeführt werden kann, weisen üblicherweise eine Aufnahme für die zellhaltige Suspension und mindestens zwei Elektroden auf, deren Elektrodenflächen zueinander ausgerichtet sind. Die Elektroden sind so angeordnet, daß sich im Betrieb der Kammer zwischen den Elektrodenflächen Suspension befindet. Werden die Elektroden nun mit unterschiedlichen Polen einer Spannungsquelle verbunden, so kann zwischen den Elektrodenflächen ein elektrisches Feld erzeugt werden, das die Elektrofusion der in der Suspension enthaltenen Zellen bewirkt.

Die Elektrofusion kann z.B. in 3 Schritten verlaufen. In einem ersten Schritt (Pre-Alignment) werden die Zellen mittels Dielektrophorese miteinander in Kontakt gebracht. In einem zweiten Schritt (Puls) wird ein Puls gegeben, der den Durchbruch der Zellmembranen zur Folge hat, worauf die Zellmembranen und damit die Zellen fusionieren können. Um die Fusionspartner zu stabilisieren, können in einem dritten Schritt (Post-Alignment) die aufgebrochenen Zellen solange mittels Dielektrophorese miteinander in Kontakt gehalten werden, bis die Fusion abgeschlossen ist.

Die Abfolge dieser Schritte ist nicht zwingend. Denkbar ist auch, daß nur der Puls gegeben wird und dann ggf. zur Stabilisierung der Fusion ein Zentrifugationsschritt durchgeführt wird.

Eine umfassende Beschreibung der Elektrofusion kann z.B. dem Buch von U. Zimmermann & G. Neill ("Electro manipulation of cells", CRC Pub., Bocaration Florida, 1996) entnommen werden.

Bei der Elektrofusion ist zu beachten, daß das in der Kammer erzeugte elektrische Feld inhomogen ist. Nur in einem inhomogenen Feld kommt es zu dem gewünschten Alignment der Zellen mittels Dielektrophores jeweils im Bereich der höchsten Feldstärke.

Es sind unterschiedliche gattungsgemäße Vorrichtungen bekannt, mit denen sich inhomogene Felder erzeugen lassen. Die DE 3317415 beschreibt eine Kammer, in die ein z.B. zylindrischer Kern eingesetzt ist, um den drahtförmige Elektroden in Form von z.B. einer Doppelhelix gewickelt sind. Mit dieser Kammer lassen sich gute Fusionsergebnisse erzielen. Allerdings können nur geringe Suspensionsvolumina verarbeitet werden, die in manchen Anwendungen, z.B. bei der oben angesprochene Krebstherapie nicht ausreichend sind.

Aus der US 4,578,168 ist eine Kammer bekannt, in die mehrere flächige Gitterelektroden eingesetzt sind. Nachteilig an diesen Elektroden ist, daß sich die Zellen in den Gitterstrukturen verfangen und sich nach Abschluß der Fusion nur schwierig entfernen lassen.

Die oben angesprochene Veröffentlichung in Nature Medicine Vol. 6 (2000) beschreibt die Verwendung einer Elektropurationsküvette, deren Elektroden mit einem dielektrischen Wachs beschichtet wurden, um das gewünschte inhomogene Feld zu erzeugen. Nachteilig ist hier die fehlende Möglichkeit zur Standardisierung bzw. Reproduzierbarkeit.

Aufgabe der Erfindung ist es, ausgehend von dem Stand der Technik eine Kammer insbesondere für die Elektrofusion zu schaffen, die gegenüber dem Stand der Technik die Verarbeitung großer Suspensionsvolumina unter gut definierten Bedingungen erlaubt.

Gelöst wird die Erfindung mit einer Kammer, die die kennzeichnenden Merkmale des Anspruches 1 aufweist.

Die Erfindung deckt damit im wesentlichen Kammern ab mit plattenförmigen geschlossenflächigen Elektroden, die Elektrodenflächen aus elektrisch leitendem Material aufweisen, wobei die Elektrodenfläche mindestens einer der beiden Elektroden so geformt ist, daß zwischen beiden Elektroden ein inhomogenes Feld mit mehreren über die Elektrodenfläche verteilten Maxima erzeugbar ist.

Die Erfindung soll dabei sowohl Elektroden mit geschlossenflächigen Elektrodenflächen abdecken als auch Elektroden, in denen die Elektrodenfläche nur partiell in den Bereichen der Elektrode ausgebildet ist, in denen die gewünschten Feldstärkemaxima erzeugt werden sollen. Denkbar ist z.B. in diesem Zusammenhang eine Elektrode, die einen isolierenden Kern aufweist, der partiell mit z.B. streifenförmigen Beschichtungen aus elektrisch leitendem Material versehen ist, die gemeinsam kontaktiert werden.

Ein wesentlicher Vorteil der Erfindung ist, daß sich die geschlossenflächigen Elektroden der erfindungsgemäßen Kammer im Gegensatz zu den bekannten Gitterelektroden gut abspülen lassen. Es ist also ohne Probleme möglich, die gebildeten Fusionsprodukte aus der erfindungsgemäßen Kammer nach Abschluß der Elektrofusion zu entfernen.

Vorteilhafte Ausgestaltungen der Erfindung sind in den Unteransprüchen angegeben.

In einer Ausgestaltung der Erfindung ist vorgesehen, daß in mindestens einer der Elektrodenflächen der beiden Elektroden Erhebungen ausgebildet sind, die jeweils Bereiche definieren, in denen der Abstand zu der zugewandten anderen Elektrodenfläche geringer ist als in umgebenden Bereichen. Im Bereich dieser Erhebungen hat das zwischen den beiden Elektrodenflächen erzeugbare Feld jeweils maximale Werte und hier ordnen sich dann bei der Elektrofusion die Zellen aneinander an.

Diese Erhebungen können grundsätzlich beliebige Formen, z.B. Pyramiden- oder Halbkugelform etc., einnehmen. In einer weiteren besonders bevorzugten Ausgestaltung ist vorgesehen, daß die Elektrodenfläche eine gewellte Form aufweist. Im Bereich der Wellenberge ergeben sich dann die gewünschten Feldstärkemaxima unterbrochen von niedrigeren Feldstärkewerten im Bereich der angrenzenden Wellentäler.

Denkbar ist, daß die Erhebungen, z.B. die Wellenform, nur in der einen Elektrodenfläche ausgebildet sind und die andere Elektrodenfläche eben ist. Eine weitere vorteilhafte Ausgestaltung der Erfindung sieht jedoch vor, daß beide einander zugewandten Elektrodenflächen mit gegenüberliegenden aufeinander zuweisenden Erhebungen ausgestattet sind. In diesem Fall erhält man besonders klar definierte Bereiche mit deutlich höherer Feldstärke als in den umgebenden Bereichen.

Für den Fall, daß gewellte Elektrodenflächen eingesetzt werden, kann vorgesehen werden, daß bei beiden einander zugewandten Elektrodenflächen die Wellenberge und Wellentäler gleichgerichtet verlaufen. Denkbar ist aber auch, daß Wellenberge und Wellentäler im Winkel zueinander ausgerichtet werden. Man erhält so eine größere Anzahl von Bereichen mit Feldstärkemaxima, was in einigen Anwendungen von Vorteilen sein kann.

Im Hinblick auf eine gute Abspülbarkeit kann weiterhin vorgesehen sein, daß die Oberfläche der Elektrodenflächen partiell mit einem nicht elektrisch leitenden Material beschichtet und dabei so nivelliert wird, daß nur die Bereiche, die die gewünschten Feldstärkemaxima erzeugen, frei bleiben. Im Fall der gewellten Elektrodenfläche wäre es z.B. denkbar, die Wellentäler entsprechend aufzufüllen, so daß die beschichtete Elektrodenfläche eine annähernd ebene Oberfläche erhält, von der sich die Zellen bzw. Fusionsprodukte gut abspülen lassen.

Die Elektroden können vollständig aus elektrisch leitfähigem Material hergestellt sein. Denkbar ist aber auch, daß die Elektroden einen Kern aus anderem Material aufweisen und nur die Elektrodenfläche in Form einer Beschichtung aus leitfähigem Material auf diesem Kern aufgebracht ist. Die Elektrodenfläche ihrerseits kann dann (wie oben erwähnt) noch mit einer weiteren partiellen Beschichtung aus nicht elektrisch leitfähigem Material versehen sein.

Insbesondere bei der Ausgestaltung, bei der die Elektrodenfläche in Form einer Beschichtung aus leitfähigem Material auf dem Kern aufgebracht ist, kann vorgesehen sein, daß die Elektrodenfläche nur partiell auf der Elektrode ausgebildet ist. Man kann so den erforderlichen Strom geringer halten als bei der Variante, in der die Elektrodenfläche geschlossenflächig vorgesehen ist, unter Beibehaltung des Vorteils der guten Abspülbarkeit.

Die Wahl der geeigneten elektrisch leitfähigen Materialien ist nicht beschränkt. Es können alle Materialien zum Einsatz kommen, die sich entsprechend verarbeiten lassen und mit denen die zur Elektrofusion erforderlichen Felder erzeugt werden können.

Bevorzugt können im Hinblick auf eine Vereinfachung des Herstellungsprozesses z.B. elektrisch leitende Kunststoffe (Kunststoffe, die mit Kohlenstoff gefüllt sind) oder Halbleitermaterialien zum Einsatz kommen. Diese Materialien lassen sich besonders einfach mittels Spritzgusstechnik verarbeiten, was z.B. insbesondere bei Kammern, die als Einmalartikel gedacht sind, von Vorteil ist. Denkbar ist aber auch, daß metallisierte Kunststoffplatten eingesetzt werden.

Bei der Kammer kann es sich wie beim Stand der Technik um eine Kammer handeln, in der mindestens zwei Elektroden mit einander zugewandten Elektrodenflächen anordenbar oder bereits fest angeordnet sind.

Denkbar ist selbstverständlich auch, daß eine ganze Reihe von z.B. parallel angeordneten Elektroden in der Kammer vorgesehen werden, die jeweils Paare von einander zugewandten Elektrodenflächen ausbilden.

Die erfindungsgemäße Kammer kann einen Deckel aufweisen, mit dem die Aufnahme für die Suspension flüssigkeitsdicht verschlossen werden kann. Denkbar ist z.B., daß die Elektroden an dem Deckel befestigt sind und mit dem Deckel zusammen von der Kammer z.B. zum Abspülen entfernt werden können. Die Verdrahtung zum Anschluß der Elektroden an die Spannungsquelle kann ebenfalls im Deckel vorgesehen sein kann.

Eine weitere vorteilhafte Ausgestaltung sieht vor, daß der Abstand der Elektroden zueinander in der Kammer variabel einstellbar ist. Denkbar ist z.B. die Elektroden in Schienen oder dergleichen zu lagern, in denen sie zu Einstellung des gewünschten Elektrodenabstandes verschoben werden können.

Die erfindungsgemäße Kammer kann als Einmalartikel ausgebildet werden. Unabhängig davon, ob sie als Einmalartikel oder für den Dauergebrauch bestimmt ist, sollte sie so beschaffen sein, daß sie sterilisierbar ist. Vorzugsweise ist die Kammer so ausgebildet, daß sie gemeinsam mit den Elektroden mittels Bestrahlung sterilisiert werden kann.

Wie oben angesprochen, kann die Elektrofusion in aller Regel in mehreren Schritten erfolgen, in denen unterschiedliche elektrische Felder an die zellhaltige Suspension angelegt werden. Denkbar ist aber auch, daß nur ein Puls gegeben wird, der das gewünschte Alignment der Zellen und mehr oder weniger gleichzeitig die Fusion bewirkt. Zur Stabilisierung der Fusionsprodukte kann dann anstelle eines elektrischen Feldes auch eine Verdichtung der Zellen mittels Schwerkraft, insbesondere durch Zentrifugation erfolgen. In diesem Zusammenhang sieht eine weitere Ausgestaltung der Erfindung vor, daß die Kammer zentrifugierbar ausgestaltet ist.

Eine letzte Ausgestaltung schließlich betrifft die zu verarbeitenden Suspensionsvolumina. Wie oben angesprochen, ist ein Ziel der Erfindung, möglichst große Suspensionsvolumina zu verarbeiten. Die erfindungsgemäße Kammer erlaubt bereits in den oben angesprochenen Ausgestaltungen die Verarbeitung von Suspensionsvolumina, die für die im Moment angedachten Anwendungen ausreichen. Falls noch größere Volumina verarbeitet werden sollen, sieht eine weitere Ausgestaltung der Erfindung vor, daß die Kammer als Durchflußkammer ausgebildet ist. In dieser Ausgestaltung weist die Kammer einen Anschluß für eine Zuleitung und einen Anschluß für eine Ableitung und ggf. innerhalb der Kammer flüssigkeitsleitende Einrichtungen auf, die einen Durchflußbetrieb erlauben.

Im folgenden soll die Erfindung anhand von zwei Abbildungen, die zwei Ausführungsbeispiele zeigen, näher erläutert werden.

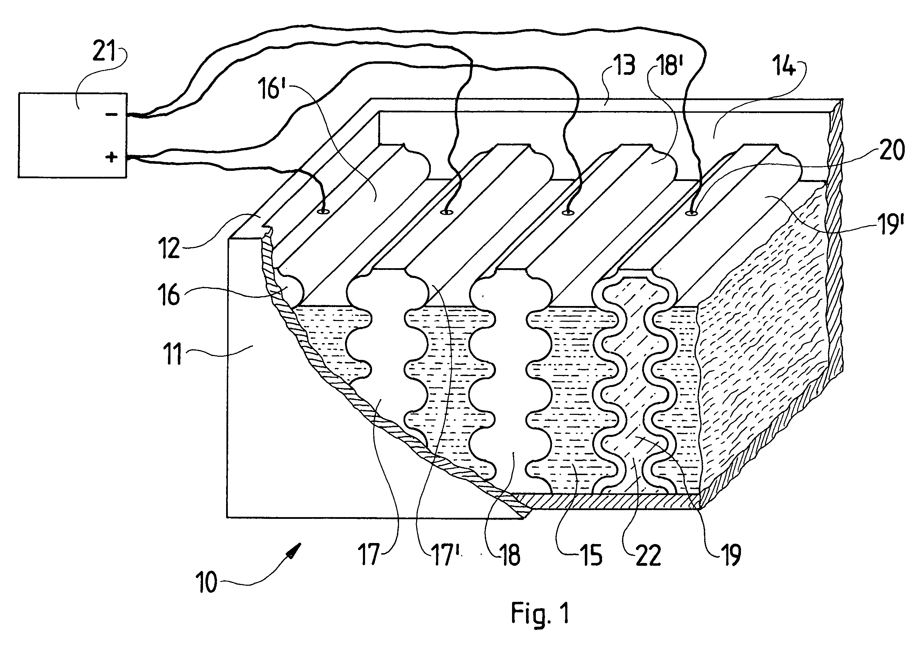

- Fig. 1: zeigt in einer Teilansicht eine Ausführungsform der erfindungsgemäßen Kammer,
- Fig. 2: zeigt eine in der erfindungsgemäßen Kammer einsetzbare Elektrode in einer weiteren Ausführungsform.

Die Figur zeigt eine Teilansicht einer Kammer 10 mit Wänden 11, 12 und 13 zur Bildung einer inneren Aufnahme 14, in die zellhaltige Suspension 15 eingefüllt ist.

Nicht dargestellt ist ein Deckel, mit dem die Kammer 10 verschlossen werden kann.

In der Kammer 10 sind Elektroden 16, 17 , 18 und 19 vorgesehen. Die dargestellten Elektroden weisen gewellte Elektrodenflächen 16', 17', 18' und 19' auf, wobei an den Elektroden 17, 18 und 19 jeweils beidseitig Elektrodenflächen ausgebildet sind.

An den Elektroden, gezeigt am Beispiel der Elektrode 19, sind Kontaktpunkte 20 vorgesehen, über die die Elektroden mit einem Pol einer Spannungsquelle 21 verbindbar sind.

Die in der Figur dargestellten Elektroden 16, 17 und 18 bestehen insgesamt aus leitfähigem Material. Die Elektrode 19 dagegen weist einen Kern 22 auf, auf dem die Elektrodenflächen 19' als Beschichtung aus leitfähigem Material vorgesehen sind.

Denkbar ist natürlich auch, daß der Kern 22 weggelassen wird und die Elektrode 19 nur die Elektrodenflächen 19' aufweist.

In der gezeigten Ausführung sind die Elektroden paarweise verdrahtet, d.h. die Elektroden 16 und 18 stehen in Verbindung mit dem einen Pol der Spannungsquelle 21 und die Elektroden 17 und 19 mit dem anderen Pol.

Die dargestellte Ausführungsform erlaubt eine besonders einfache Umsetzung der Erfindung.

Denkbar sind natürlich auch andere Ausführungen. So ist es z.B. möglich, jeweils nur eine der Elektrodenflächen wellig auszubilden und die andere eben zu gestalten. Denkbar ist natürlich auch, daß die gewellten Elektrodenflächen nicht gleichgerichtet sondern in unterschiedlichen Winkeln angeordnet werden.

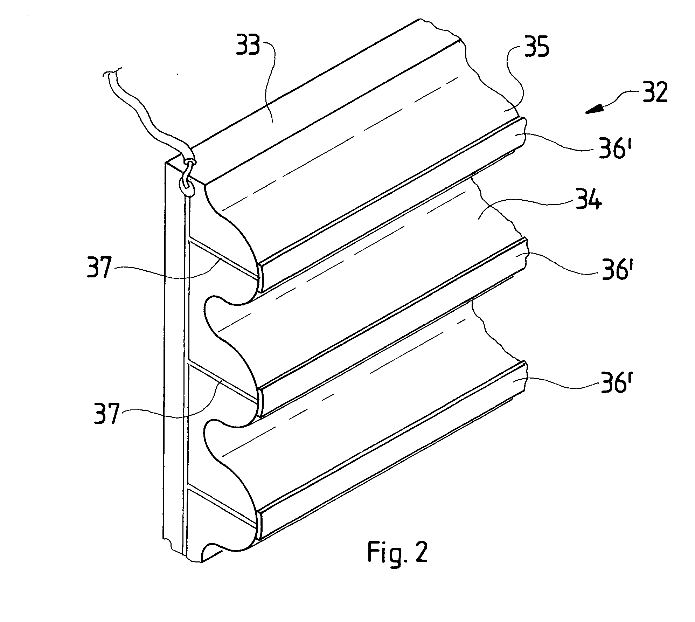

Fig. 2 zeigt eine Elektrode 32 mit einem Kern 33 aus isolierendem Material. Der Kern 33 weist eine gewellte Oberfläche 34 mit Wellenbergen 35 auf. Auf der Oberfläche 34 ist eine Elektrodenfläche in Form von partiellen Beschichtungen 36' der Wellenberge 35 vorgesehen. Zwischen den Wellenbergen 35 ist die Oberfläche 34 damit nicht elektrisch leitend. Die Elektrodenfläche bzw. ihre partiell auf der Oberfläche 34 ausgebildeten Beschichtungen 36' sind über seitliche Anschlüsse 37 gemeinsam mit einem Pol einer nicht gezeigten Spannungsquelle verbindbar.

Der Vorteil einer Elektrode in der zuletzt gezeigten Ausführung besteht darin, daß sich ein inhomogenes Feld mit definierten Maxima erzeugen läßt, wobei ein geringerer Strom angelegt werden kann, als dies bei einer geschlossenflächigen Elektrodenfläche notwendig wäre.

Denkbar wäre selbstverständlich auch, solche partiellen Beschichtungen auf einer Elektrode mit einem Kern vorzusehen, der eine ebene Oberfläche aufweist. Auch dann würde man definierte Feldstärkemaxima erzeugen können.

## Patentansprüche

1. Kammer zur Behandlung von in einer Suspension enthaltenen Zellen im elektrischen Feld, mit einer Aufnahme, in der die Suspension angeordnet werden kann und mit mindestens zwei Elektroden, deren Elektrodenflächen einander zugewandt ausgerichtet sind, wobei zwischen den Elektrodenflächen Suspension anordenbar ist, und die Elektroden mit unterschiedlichen Polen einer Spannungsquelle zur Erzeugung eines elektrischen Feldes zwischen den Elektrodenflächen verbindbar sind und wobei die Elektroden so beschaffen sind, daß ein inhomogenes Feld erzeugbar ist, **dadurch gekennzeichnet, daß** die Elektroden (16, 17, 18 und 19) plattenförmig und geschlossenflächig ausgebildet sind und Elektrodenflächen (16', 17', 18', 19') aus elektrisch leitendem Material aufweisen, wobei die Elektrodenfläche (16', 17', 18', 19'; 36') mindestens einer der beiden Elektroden (16, 17, 18, 19; 32) so geformt ist, daß zwischen beiden Elektroden (16", 17', 18', 19'; 32) ein inhomogenes Feld mit mehreren über die Elektrodenfläche verteilten Maxima erzeugbar ist.

2. Kammer nach Anspruch 1, **dadurch gekennzeichnet, daß** die Elektrodenflächen (16', 17', 18', 19') geschlossenflächig ausgebildet sind.

3. Kammer nach Anspruch 1 oder2, **dadurch gekennzeichnet, daß** die Elektrodenfläche in mehreren Bereichen Erhebungen aufweist.

4. Kammer nach Anspruch 3, **dadurch gekennzeichnet, daß** die Elektrodenfläche (16', 17', 18', 19'; 36') gewellt geformt ist.

5. Kammer nach Anspruch 4, **dadurch gekennzeichnet, daß** die zugewandten Elektrodenflächen beider Elektroden wellig geformt sind, wobei Wellentäler und Wellenberge gleichgerichtet und in beiden Flächen jeweils gegenüberliegend ausgebildet sind.

6. Kammer nach Anspruch 4 oder 5, **dadurch gekennzeichnet, daß** die Wellentäler mit einem isolierenden Material gefüllt sind.

7. Kammer nach einem der Ansprüche 1 bis 5, **dadurch gekennzeichnet, daß** die Elektroden (16, 17, 18) insgesamt aus elektrisch leitfähigem Material gebildet sind.

8. Kammer nach einem der Ansprüche 1 bis 4, **dadurch gekennzeichnet, daß** die Elektroden einen Kern (22) mit einer die Elektrodenfläche (19') bildenden Beschichtung aus leitfähigem Material aufweisen.

9. Kammer nach Anspruch 8, **dadurch gekennzeichnet, daß** die Elektrodenfläche (36) nur partiell auf dem einen Kern (22) ausgebildet ist.

10. Kammer nach einem der Ansprüche 1 bis 9, **dadurch gekennzeichnet, daß** ein Deckel zum Verschluß der Aufnahme (14) vorgesehen ist.

11. Kammer nach Anspruch 10, **dadurch gekennzeichnet, daß** mindestens ein Teil der Elektroden an dem Deckel angeordnet ist.

12. Kammer nach einem der Ansprüche 1 bis 11, **dadurch gekennzeichnet, daß** die Elektrodenflächen aus elektrisch leitfähigem Kunststoff oder Halbleitermaterialien gebildet sind.

13. Kammer nach einem der Ansprüche 1 bis 12, **dadurch gekennzeichnet, daß** sie sterilisierbar, insbesondere durch Bestrahlung sterilisierbar, ausgebildet ist.

14. Kammer nach einem der vorgehenden Ansprüche, **dadurch gekennzeichnet, daß** sie zentrifugierbar ausgebildet ist.

15. Kammer nach einem der vorhergehenden Ansprüche, **dadurch gekennzeichnet, daß** sie Einrichtungen aufweist, mit denen die Suspension in Durchfluß hindurchgeleitet werden kann.

16. Kammer nach einem der vorhergehenden Ansprüche, **dadurch gekennzeichnet, daß** sie als Einmalartikel ausgebildet ist.
